# EUROPÄISCHE PATENTSCHRIFT

(11) **EP 1 553 915 B1**
(45) Veröffentlichungstag und Bekanntmachung des Hinweises auf die Patenterteilung: **05.11.2008**
(21) Anmeldenummer: 03807742.6
(22) Anmeldetag: 18.06.2003
(51) Int. Cl.: A61K 6/08

(54) **DENTALE ABDECKMASSE FÜR ZÄHNE UND ZAHNFLEISCH**
DENTAL MASKING PRODUCT FOR TEETH AND GUM
PRODUIT DE COUVERTURE DENTAIRE POUR DENTS ET GENCIVE

(30) Priorität: 27.09.2002 DE 10245274
(43) Veröffentlichungstag der Anmeldung: 20.07.2005
(73) Patentinhaber: VOCO GmbH, 27472 Cuxhaven (DE)
(72) Erfinder: VAN EIKEREN, Andreas, 27474 Cuxhaven (DE); PLAUMANN, Manfred, Thomas, 27476 Cuxhaven (DE)
(74) Vertreter: Hauck Patent- und Rechtsanwälte
(86) Internationale Anmeldenummer: PCT/EP2003/006433
(87) Internationale Veröffentlichungsnummer: WO 2004/032883

(56) Entgegenhaltungen:
- WO-A-96/27342
- DE-A- 4 306 997
- DE-A- 19 915 004
- DE-C- 19 959 255
- US-B1- 6 305 936

## Beschreibung

Die Erfindung betrifft die Verwendung einer Abdeckmasse zur Herstellung einer Isolierung von zu behandelnder Zahnsubstanz und eines Schutzes des umgebenden Zahnfleisches.

Bei einigen zahnärztlichen Behandlungsverfahren werden im Mund aggressive chemische Substanzen eingesetzt, die Verätzungen an der Mundschleimhaut hervorrufen können. Dazu zählen die Säure-Ätz-Technik im Rahmen einer Füllungslegung mit einem Komposit und das Bleichen mit hochprozentigen Peroxidpräparaten beim Zahnarzt (in office bleaching). Bei der Säure-Ätz-Technik wird der Zahnschmelz innerhalb einer präparierten Zahnkavität zur Verbesserung der Adhäsion vor dem Auftragen eines Primers und/oder Bondings mit einer hochkonzentrierten, üblicherweise etwa 35 Gew.-%igen Phosphorsäure behandelt. Dabei läßt man die Phosphorsäurelösung oder das Phosphorsäuregel für ca. 30 Sekunden auf den Zahnschmelz einwirken. Insbesondere bei Präparationen, deren Ränder nahe am Zahnfleisch oder einem Nachbarzahn liegen, ist ein Schutz dieser Gewebe vor dem Ätzmittel notwendig. Es besteht ferner der Wunsch, den behandelten Zahn oder gleich mehrere Zähne gegenüber dem umgebenden Gewebe des Mundraums flüssigkeitsdicht abzuschirmen, um beispielsweise den Zutritt von Blut oder Speichel zum behandelten Zahn zu verhindern.

Bei der "in office" Bleichtherapie werden zum Aufhellen der Zähne Bleichmittel mit einem Gehalt von bis zu 35 Gew.-% Wasserstoffperoxid direkt auf die Zahnoberflächen aufgetragen. Insbesondere bei der Aufhellung vitaler Zähne wird das Bleichmittel auf die äußeren Zahnoberflächen bis nahe an den Zahnfleischsaum heran appliziert. Um Verätzungen vorzubeugen, ist auch hier eine schützende Abdeckung der Mundschleimhaut unumgänglich.

Eine Möglichkeit der Abschirmung besteht in der Verwendung eines Spanntuchs aus Gummi, das als Kofferdam bezeichnet wird. Dabei muß der Zahnarzt das Tuch an den passenden Stellen perforieren und Löcher entsprechender Größe in das Tuch stanzen, durch die anschließend die zu behandelnden Zähne hindurch gesteckt werden. Bei unpassender Lochgröße oder an Einfallstellen an der Zahnoberfläche besteht häufig das Problem, dass das Spanntuch nicht genau oder nicht stramm genug entlang des Zahnfleischsaums sitzt und abdichtet und damit die Mundschleimhaut nicht ausreichend schützt. Das Legen eines Kofferdams wird von vielen Zahnärzten als zu aufwändig und umständlich angesehen. Es besteht die Gefahr, dass das Gummi beim Spannen oder während der Behandlung reißt oder sich löst. Hierbei geht die isolierende Wirkung des Kofferdams verloren und die auf der Außenseite des Gummituchs befindlichen Stoffe können in die Mundhöhle gelangen. Von den Patienten wird die sperrige Apparatur und das Befestigen des Gummis an den Zahnhälsen als unangenehm empfunden.

In der US-6305936 werden Zusammensetzungen und Verfahren angegeben, die die oben benannten Nachteile der Kofferdamtechnik bei dem Abdichten von Weichgewebeteilen im Mundinnenraum überwinden sollen. In der Patentschrift wird ein polymerisierbares Material geschützt, das wenigstens ein Monomer, ein Härtungsmittel und wenigstens eine weitere Verbindung umfaßt. Bei den Zusammensetzungen handelt es sich um herkömmliche, lichthärtende Acrylatsysteme, die zur Anwendung auf Weichgewebe erheblich modifiziert werden müssen. Da die durch Belichtung initiierte radikalische Polymerisation von Acrylaten bei der Vernetzung eine große Wärmermenge freisetzt, muss bei einer Anwendung dieser Systeme auf Mundweichgewebe, wie beispielsweise dem Zahnfleisch, ein nichtreaktives Additiv wie Mineralöl oder Polyol der Mischung zugesetzt werden, um sicherzustellen, daß das Gewebe des Patienten keine Verbrennungen erleidet. Der Zusatz eines Reichmachers ist zudem deshalb notwendig, um die Vernetzungsdichte des Acrylats zu reduzieren und so gewährleisten zu können, dass das polymerisierte Material nach der Behandlung wieder vom Gewebe entfernt werden kann. Zusätzlich soll durch Beigabe eines reflektierenden Materials wie beispielsweise Glimmer ein Teil der durch die Polymerisationslampe in die Mischung eingestrahlte Energie reflektiert werden, um so einen Beitrag zur Reduzierung der in der Mischung resultierenden Wärmeenergie zu leisten.

Des weiteren wird in der Patentschrift die Zugabe eines Verdickungsmittels wie beispielsweise Xanthan, Cellulosederivat, Carboxypolymethylen, Polyethylenoxid oder hochmolekulares Polypropylenglykol zur polymerisierbaren Masse empfohlen, um ihr mukoadhäsive Eigenschaften zu verleihen.

Man sieht, dass nur ein außergewöhnlich hoher Aufwand in der Modifizierung des Acrylatsystems imstande ist, eine Formulierung zustande zu bringen, die den gröbsten Kriterien zur Anwendung der Rezeptur auf Weichgewebe entspricht. Weiterhin äußerst nachteilig an der Erfindung ist der Umstand, daß durch Einsatz eines Weichmachers versucht wird, den Grad der Polymerisation wesentlich abzusenken. Ein reduzierter Polymerisationsgrad ermöglicht das Vorliegen entweder freier Monomere oder von entsprechenden oligomeren Struktureinheiten geringeren Molekulargewichts. Es ist bekannt, dass polymere Verbindungen mit einem Molekulargewicht von weniger als 1000 g/mol leicht biologische Membranen passieren und bioakkumulieren (J.H.Hamilton, R.Sutcliffe, Ecological Assessment of Polymers, 1996, Seite 274) . Im Falle der stark toxischen Acrylatkomponenten bis-GMA oder HEMA sollte ein verminderter Polymerisationsgrad dementsprechend zu zytotoxischen Problemen bei Anwendung der Erfindung führen. Ein weiterer Nachteil ist die zeitaufwendige Anwendung durch Bestrahlung mit einer Polymerisationslampe.

Hierbei muß, Stück für Stück, je nach Breite des Lichtaustrittsfensters 20 Sek. pro Stück polymerisiert werden.

Die WO 96/27342 A und die DE 199 15 004 A1 offenbaren Abformmaterialien auf Silikonbasis zum Herstellen eines Abdruckes der Zähne. Das Abformmaterial wird mit Hilfe eines Löffels appliziert und nach dem Erstarren abgenommen. Gemäß WO 96/27342 A wird ein Grobabdruck der Zähne erneut mit einem leicht fließfähigen Material und einem blutstillenden Mittel gefüllt und auf den Zähnen plaziert, um das blutstillende Mittel dem Zahnfleischsaum gezielt zuzuführen. Gemäß DE 199 15 004 A1 können zu abzuformenden Stellen des Kiefers zunächst mit Abformmasse umspritzt werden. Danach wird ein mit Abformmasse befüllter Löffel aufgesetzt und nach dem Herausnehmen die negative Form der Kiefersituation erhalten.

Davon ausgehend liegt der Erfindung die Aufgabe zugrunde, eine Isolierung von zu behandelnder Zahnsubstanz und einen Schutz für umgebendes Zahnfleisch bereitzustellen, die bzw. der schneller und bequemer anwendbar und weniger toxisch ist.

Die Aufgabe wird durch die Verwendung einer Abdeckmasse mit den Merkmalen des Anspruches 1 gelöst. Vorteilhafte Ausgestaltungen der Erfindung sind in den Unteransprüchen angegeben.

Es wurde gefunden, dass bei Umgebungstemperatur selbsthärtende Systeme, die zu einem elastomeren Material aushärten, die gestellte Aufgabe sehr gut erfüllen. Bevorzugt handelt es sich dabei um Systeme, die direkt vor dem und/oder beim Applizieren aus mehreren Komponenten, bevorzugt aus zwei Komponenten, zu einer homogenen Masse gemischt werden. Als Umgebungstemperatur ist hier der Bereich zwischen üblicher Raumtemperatur (15-25°C) und Mundtemperatur (ca. 37°C) zu verstehen. Die Aushärtung startet erfindungsgemäß in diesem Temperaturbereich spontan direkt nach dem Mischen der Komponenten, ohne dass zusätzliche Energiezufuhr durch Licht oder Wärme notwendig ist.

Als besonders geeignet stellte sich die Substanzklasse der sogenannten "kalthärtenden", additionsvernetzenden Silikone (RTV-2 A-Silikone) heraus. Das Material wird bevorzugt als Paste im Mund aufgetragen und geht durch die Vernetzung von einer streichfähigen Konsistenz in den gummielastischen Zustand über. Das vernetzte Material haftet überraschenderweise am Zahnfleisch und kann aufgrund seines elastomeren Charakters nach der Behandlung in einem Stück leicht von der Unterlage abgezogen und aus dem Mund entfernt werden. Die für den erfinderischen Zweck ausgezeichnete Zahnfleischhaftung des Silikons, die schon bei relativer Trockenlegung des Weichgewebes nur mit Hilfe von Watterolle und Luftbläser beobachtet wurde, stellt einen völlig unerwarteten Befund dar, da eine ausgeprägt starke Hydrophobie eine allgemeine und kennzeichnende Eigenschaft der Silikone ist. Unter starker Hydrophobie versteht man den Effekt, dass Wasser in flüssiger Phase die Phasengrenzfläche Silikon/ Luft nicht oder nur relativ wenig benetzt. Da das Zahnfleisch, wie alle anderen Weichgewebeteile des Mundinnenraums, permanent mit einem charakteristischen Feuchtigkeitsfilm überzogen sind, sollte man erwarten, dass ein hydrophobes Material aufgrund fehlender Benetzung keinerlei Haftung auf dem Weichgewebe zeigt.

Die Hydrophobie der ausgehärteten Oberflächen der Silikone unterstützt die abdichtende und abdeckende Wirkung gegenüber den üblichen hydrophilen Behandlungsmitteln wie Bleich- und Ätzmitteln. Überdies sind sie geschmacks- und geruchsneutral, physiologisch unbedenklich und auf sehr kurze Abbindezeiten einstellbar. Sie zeigen im Gegensatz zu den Acrylaten, der Substanzklasse aus dem Stand der Technik, keine nennenswerte Exothermie während der Härtung und keinen messbaren Dimensionsschwund.

Weitere geeignete Substanzklassen als Abdeckmasse zur Herstellung einer Isolierung von zu behandelnder Zahnsubstanz und eines Schutzes des umgebenden Zahnfleisches und/oder benachbarter Zähne vor dentalen Behandlungsmitteln stellen die kondensationsvernetzenden Silikone (C-Silikone) und Polyethermaterialien, wie sie in der DE 4306997 beschrieben sind, dar.

Das Mischen der Komponenten erfolgt entweder manuell auf einem Mischblock mit einem Spatel oder vorzugsweise automatisch mit Hilfe eines Kartuschensystems mit statischem Mischrohr zur Direktapplikation. Bei der bevorzugt eingesetzten Direktapplikation der Silikonmassen müssen die beiden Komponenten zunächst in eine Doppelkammerkartusche abgefüllt werden. Die Applikation erfolgt dann durch Auspressen der Pasten aus der Doppelkammerkartusche mit Hilfe eines Doppelstempels durch eine statische Mischkanüle auf das zu schützende Gewebe des Mundinnenraums. Eine unterschiedliche Einfärbung der beiden ungemischten Ausgangsmassen erlaubt nach dem Durchgang der Pasten durch die Mischkanüle anhand des resultierenden Farbtons die Homogenität der Mischung zu überprüfen. Die chemische Härtung beginnt direkt nach dem Auftrag der Pasten auf das Gewebe und ist innerhalb kurzer Zeit abgeschlossen.

Das Mischungsverhältnis der beiden Komponenten kann zwischen 1:10 und 10:1 betragen und liegt bevorzugt zwischen 1:4 und 4:1 und besonders bevorzugt zwischen 2:1 und 1:2.

Werden die erfindungsgemäßen Abdeckmassen in einer Doppelkammerkartusche mit statischer Mischkanüle zur substanzundurchlässigen Abdeckung von Zahn und/oder Zahnfleisch im Mundinnenraum eingesetzt, so zeigen sich die vorteilhaften Eigenschaften dieses Anwendungsverfahrens:
Aufgrund ihrer einstellbaren rheologischen Eigenschaften sind diese leicht und punktgenau applizierbar, sie zeigen ein gutes Anfließverhalten und sorgen so für eine gute Abdichtung, insbesondere am Zahnfleischsaum (Zahnhals, Papillen). Konsistenz und ausreichend lange Verarbeitungszeit ermöglichen Korrekturen während des Auftrags der Pasten und gewährleisten, dass das Material während der Applikation nicht bereits in der Mischkanüle aushärtet. Nach dem Auftragen erfolgt eine rasche Aushärtung im Mund, so dass direkt nach der Applikation mit der weiteren Behandlung fortgefahren werden kann, ohne dass zwischendurch eine Wartezeit oder ein zusätzlicher Arbeitsschritt wie eine Lichthärtung notwendig wären.

Bei den additionsvernetzenden Silikonen erfolgt die Härtung in der Regel durch eine platinkatalysierte Additionsreaktion von SiH-funktionellen Polysiloxanen an Polysiloxane mit ungesättigten Kohlenwasserstoffgruppen, in der Regel Vinyl- oder Allylgruppen. Die Silikonmassen werden üblicherweise als Zweikomponentensystem zum Einsatz gebracht. Der Aufbau der einzelnen Komponenten erfolgt in einer Weise, dass jede Komponente für sich nicht-reaktiv und somit stabil ist. Die Lagerstabilität der Silikonmassen wird hierbei durch eine Trennung von SiH funktionellem Polysiloxan und Platinkatalysator gewährleistet. In der Regel enthält die eine Komponente ein vinylfunktionelles Polydimethylsiloxan, Füllstoff und ein SiHfunktionelles Polydimethylsiloxan, während die andere Komponente ebenfalls ein vinylfunktionelles Polydimethylsiloxan, Füllstoff und einen Platinkatalysator enthält. Über die Füllstoffgehalte und über die Viskositäten der eingesetzten Polysiloxane läßt sich die Fließfähigkeit der Pasten steuern. Bei der Verwendung eines Kartuschensystems zum automatischen Dosieren und Mischen der Komponenten ist auf eine gute Auspressbarkeit und Mischbarkeit der Komponenten zu achten, was vorteilhaft durch ähnliche Viskositäten der Polysiloxanmischungen und ähnliche Füllstoffgehalte in den Komponenten erreicht werden kann. Zusätzlich können Thixotropierungsmittel, Pigmente, Farbstoffe, Weichmacher, Stabilisatoren, Emulgatoren, Hydrophilierungsmittel, Reaktionsverzögerer, Wasserstoffabsorber oder Haftmittel als Additive eingesetzt werden. Die in den Zusammensetzungen eingesetzten Füllstoffe können saugfähige Eigenschaften (wie Kieselgur, Zeolithe, Kalziumkarbonat), materialverstärkende Eigenschaften (Kieselsäuren) oder materialnichtverstärkende Eigenschaften (Quarz, Cristobalit, Aluminiumoxid, Zinkoxid) aufweisen. Thixotropierungsmittel wie pyrogene Kieselsäuren, polymere Polyalkylenoxide oder Cellulosederivate steuern die Standfestigkeit der Mischung, während Weichmacher (u.a. Paraffine, Talg, Wachs), Stabilisatoren, Emulgatoren und Tenside für die richtigen Handhabungs- und Stabilitätseigenschaften sorgen können. Hydrophilierungsmittel sorgen für eine bessere Benetzbarkeit der Mischung auf dem feuchten Weichgewebe des Mundes und rufen ein besseres Fließverhalten der Pasten hervor. Geeignete Additive sind ethoxylierte Fettalkohole sowie die Ester oder Ether geeigneter Polyalkylenglykole.

Die vinylfunktionellen Polydimethylsiloxane sind bevorzugt linear aufgebaut und weisen Endgruppen aus Dimethylvinylsiloxaneinheiten auf. Soll ein hoher Vernetzungsgrad erreicht werden, sollten Verbindungen eingesetzt werden, die mehr als 2 Vinylgruppen pro Molekül aufweisen, wobei die Vinylgruppen dann auch über das Molekülgerüst verteilt sein können. Der Vernetzer ist ein Organohydrogenpolysiloxan mit mindestens drei SiH-Gruppen pro Molekül. Zusätzlich können auch Organohydrogenpolysiloxane mit zwei SiH-Gruppen pro Molekül als sogenannte Kettenverlängerer zur Beeinflussung des Aushärteverhaltens und der mechanischen Festigkeit eingesetzt werden. Die Mengenverhältnisse von vinylfunktionellem Polydimethylsiloxan zu dem Organohydrogensiloxan werden in der Regel so gewählt, dass pro Mol an ungesättigter Gruppe 1 bis 3 Mol an SiH-Einheiten zur Verfügung stehen.

Der eingesetzte Katalysator muss die Reaktion zwischen der SiH-Gruppe und dem Vinylsiloxan katalysieren. Es ist bekannt, zu diesem Zwecke Verbindungen des Platins oder Palladiums einzusetzen. Ein vielfach benutzter Katalysator ist ein Platinkomplex, der aus Hexachloroplatinsäure durch Reduktion mit Tetramethyldivinyldisiloxan hergestellt wird und in einer Menge von 4 bis 400 ppm, bevorzugt zwischen 20 und 50 ppm berechnet als elementares Platin und bezogen auf das Gesamtgewicht der Mischung, eingesetzt wird.

### Beispiel

### Komponente 1 (Basispaste):

23 Teile eines vinylendgestoppten Polydimethylsiloxans mit einer Viskosität von 1000 mPas, 3 Teile eines SiH-gruppenhaltigen Polydimethylsiloxans mit einem SiH-Gehalt von 4,2 mmol/g, 4 Teile einer oberflächenbehandelten pyrogenen Kieselsäure (Aerosil R 972, Degussa AG) und 0,1 Teile eines gelben Farbpigments (Sicomet S10, BASF) werden in einem Kneter homogen gemischt.

### Komponente 2 (Katalysatorpaste):

26 Teile eines vinylendgestoppten Polydimethylsiloxans mit einer Viskosität von 1000 mPas, 0,15 Teile einer Lösung eines Komplexes aus Platin und Divinyldimethylsiloxan, der 1,0 Gew-% Platin in einem vinylendgestoppten Polydimethylsiloxan mit einer Viskosität von 1000 mPas enthält, 4 Teile einer oberflächenbehandelten pyrogenen Kieselsäure (Aerosil R 972, Degussa AG) und 0,1 Teile eines weißen Farbpigments (Titandioxid) werden in einem Kneter homogen vermischt.

Basis- und Katalysatorpaste werden jeweils in eine Kammer einer 5 ml-Doppelspritze mit einem Volumenverhältnis von 1:1 (Mixpac Systems AG) blasenfrei gefüllt. Die Applikation erfolgt nach Abhalten der Lippe des Patienten und kurzer Trocknung des Applikationsfeldes mittels Luftbläser durch einen passenden Mischaufsatz direkt auf die Mundschleimhaut in der direkten Umgebung des Behandlungsfeldes. Die Paste tritt homogen gemischt aus dem Mischaufsatz in einer Konsistenz aus, die ein leichtgängiges Umspritzen des zu behandelnden Zahnes ermöglicht und ein Anfließverhalten zeigt, das eine sichere Abdichtung und Halt auf der Mundschleimhaut gewährleistet. Nach der kurzen Anfließphase weist das Material eine Standfestigkeit auf, die ein Herunterlaufen oder Spreiten des Materials verhindert und eine punktgenaue Applikation ermöglicht. Das Abbindeverhalten ermöglicht bei einem zügigen Auftrag das fortlaufende Umspritzen einer ganzen Zahnreihe ohne Verstopfen der Mischkanüle, wobei während des Auftragens die Paste mit der Kanülenspitze noch bewegt und leicht verstrichen werden kann. Die Aushärtung erfolgt bei Mundtemperatur etwa 5 Sekunden nach dem Mischen und ist innerhalb von 10 Sekunden so weit fortgeschritten, daß die Masse gummielastisch erstarrt ist und direkt mit der weiteren Behandlung begonnen werden kann. Nach der Behandlung läßt sich das ausgehärtete Silikon leicht in einem Stück rückstandsfrei entfernen.

## Patentansprüche

1. Verwendung einer Abdeckmasse umfassend ein Mehrkomponentensystem, das gemischt bei Umgebungstemperatur auf dem zu schützenden Gewebe im Mundraum selbsthärtend vernetzt, ein elastomeres, am Zahnfleisch anhaftendes Material ergibt und aus der Gruppe der A- oder C-Silikone ausgewählt ist zur Herstellung einer Isolierung von zu behandelnder Zahnsubstanz und eines Schutzes des umgebenden Zahnfleisches vor dentalen Behandlungsmitteln.

2. Verwendung nach Anspruch 1 zum Schutz von der zu behandelnden Zahnsubstanz benachbarten Zähnen vor dentalen Behandlungsmitteln.

3. Verwendung nach Anspruch 1 oder 2, bei der die Abdeckmasse durch Mischen eines Zweikomponentensystems hergestellt wird.

4. Verwendung nach einem der Ansprüche 1 bis 3, bei der die Abdeckmasse direkt nach dem Mischen bei der Applikation im Mund ein rheologisches Anfließverhalten und innerhalb einer Sekunde nach der Applikation eine solche Standfestigkeit aufweist, dass ein Herunterlaufen oder Spreiten der applizierten Masse nicht auftritt.

5. Verwendung nach einem der Ansprüche 1 bis 4, bei der die Vernetzung der Abdeckmasse innerhalb von 20 Sekunden nach dem Mischen der Komponenten beginnt und die innerhalb von 40 Sekunden nach dem Mischen der Komponenten so weit fortgeschritten ist, dass die Masse gummielastisch erstarrt ist.

6. Verwendung nach einem der Ansprüche 1 bis 5, bei der die Vernetzung der Abdeckmasse innerhalb von 10 Sekunden nach dem Mischen der Komponenten beginnt und die innerhalb von 20 Sekunden nach dem Mischen der Komponenten so weit fortgeschritten ist, dass die Masse gummielastisch erstarrt ist.

7. Verwendung nach einem der Ansprüche 1 bis 6, bei der die Vernetzung der Abdeckmasse innerhalb von 5 Sekunden nach dem Mischen der Komponenten beginnt und die innerhalb von 10 Sekunden nach dem Mischen der Komponenten so weit fortgeschritten ist, dass die Masse gummielastisch erstarrt ist.

8. Verwendung nach einem der Ansprüche 1 bis 7, bei der die Abdeckmasse im vernetzten Zustand leicht in einem Stück rückstandsfrei aus dem Mund entfernbar ist.

## Claims

1. Use of a masking product comprising a multi-component system that, mixed at ambient temperature, cross-links self-curing on the tissue to be protected in the oral cavity, yields an elastomer material adhering on the gum, and is selected from the group of A-silicones or C-silicones, for producing an isolation of tooth material to be treated and a protection of the surrounding gum from dental treatment means.

2. The use according to Claim 1 for protecting teeth adjacent to the tooth material to be treated from the dental treatment means.

3. The use according to Claim 1 or 2, in which the masking product is produced by mixing a two component system.

4. The use according to one of the Claims 1 to 3, in which the masking product directly after mixing has a rheological flow behavior during the application in the mouth, and within a second after application has a stability such that a run-down or spreading of the applied product does not occur.

5. The use according to one of the Claims 1 to 4, in which the cross-linking of the masking product begins within 20 seconds after mixing the components, and which progresses so far within 40 seconds after mixing the components that the product is hardened elastically.

6. The use according to one of the Claims 1 to 5, in which the cross-linking of the masking product begins within 10 seconds after mixing the components, and which progresses so far within 20 seconds after mixing the components that the product is hardened elastically.

7. The use according to one of the Claims 1 to 6, in which the cross-linking of the masking product begins within 5 seconds after mixing the components, and which progresses so far within 10 seconds after mixing the components that the product is hardened elastically.

8. The use according to one of the Claims 1 to 7, in which the masking product in the cross-linked state can be easily removed in one piece, residue-free, from the mouth.

## Revendications

1. Utilisation d'un produit de couverture comprenant un système à plusieurs composants qui s'autopolymérise sur le tissu à protéger de la zone buccale lorsqu'il est mélangé à température ambiante, produit un matériau élastomère adhérant sur la gencive, et est sélectionné dans le groupe des silicones A ou C pour la fabrication d'un isolant de la substance dentaire devant être traitée et d'une protection de la gencive environnante contre les moyens de soin dentaire.

2. Utilisation selon la revendication 1 pour protéger les dents adjacentes à la substance dentaire à traiter contre les moyens de soin dentaire.

3. Utilisation selon les revendications 1 ou 2, dans laquelle le produit de couverture est fabriqué par mélange d'un système à deux composants.

4. Utilisation selon une des revendications 1 à 3, dans laquelle le produit de couverture présente une fluidité rhéologique directement après le mélange lors de l'application dans la bouche, et une rigidité en l'espace d'une seconde après l'application, de sorte que le matériau appliqué ne s'écoule pas ni ne s'étale.

5. Utilisation selon une des revendications 1 à 4, dans laquelle la réticulation du produit de couverture débute dans les 20 secondes qui suivent le mélange des composants et se poursuit au cours des 40 secondes qui suivent le mélange des composants, jusqu'à ce que le matériau durcisse tout en restant caoutchouteux.

6. Utilisation selon une des revendications 1 à 5, dans laquelle la réticulation du produit de couverture débute dans les 10 secondes qui suivent le mélange des composants et se poursuit au cours des 20 secondes qui suivent le mélange des composants, jusqu'à ce que le matériau durcisse tout en restant caoutchouteux.

7. Utilisation selon une des revendications 1 à 6, dans laquelle la réticulation du produit de couverture débute dans les 5 secondes qui suivent le mélange des composants et se poursuit au cours des 10 secondes qui suivent le mélange des composants, jusqu'à ce que le matériau durcisse tout en restant caoutchouteux.

8. Utilisation selon une des revendications 1 à 7, dans laquelle le produit de couverture peut être retiré facilement de la bouche à l'état réticulé d'un seul tenant sans laisser de résidus.
